# EUROPEAN PATENT APPLICATION

(11) **EP 4 442 122 A1**
(43) Date of publication of application: **09.10.2024**
(21) Application number: 23166948.2
(22) Date of filing: 06.04.2023
(51) Int. Cl.: A23G 1/00, A23G 1/30, A23G 1/32, A23L 33/11

(54) **COCOA EXTRACTION METHODS AND EXTRACTS OBTAINED BY THE SAME**

(71) Applicant: ODC Lizenz AG, 6370 Stans (CH)
(72) Inventor: Colombi, Renato, 8807 Freienbach (CH); Beeler, Nicole, 8807 Freienbach (CH)
(74) Representative: Grünecker Patent- und Rechtsanwälte PartG mbB

(57) **Abstract**

A method for obtaining extracts from cocoa fruit constituents is described, which comprises the steps of: a) subjecting cocoa fruit constituents to wet grinding in a polar solvent and separating a liquid phase from the wet ground mixture to obtain a hydrophilic extract; b) optionally separating residual solids from the hydrophilic extract; c) adsorbing the hydrophilic extract on a non-ionic macroporous resin; d) desorbing the hydrophilic extract from the non-ionic macroporous resin with a water-miscible organic solvent to obtain a polyphenolic extract; and e) concentrating the polyphenolic extract to obtain a polyphenolic concentrate. In further aspects, extracts obtained by the aforementioned process and food compositions, food supplements, medical compositions or cosmetic compositions comprising the aforementioned extracts are described.

## Description

### FIELD OF INVENTION

This invention relates to improved methods for obtaining polyphenol, theobromine and protein extracts from cocoa fruit constituents using phase separation and adsorption/desorption techniques. In certain embodiments, this invention relates to extracts obtained by the aforementioned methods and compositions comprising the same.

### BACKGROUND OF THE INVENTION

Cocoa (*Theobroma* cacao *L.*) is widely recognized as an important source of healthy ingredients, including minerals, vitamins and especially polyphenols (e.g. catechins, flavanol glycosides, anthocyanins and procyanidins). The principal polyphenol compounds present in cocoa beans are monomeric flavonoids such as (+)-catechin, (-)-epicatechin and oligomeric flavonoids such as proanthocyanidins, which are known to reduce the risk of cardiovascular events, exert blood pressure lowering activity, antiplatelet, anti-inflammatory, metabolic and anti-atherosclerotic effects, and also improve endothelial function (see A. C. Aprotosoaie et al., Diseases 2016, 4(4), 39).

Since cocoa beans are one of the best-known sources of dietary polyphenols, a number of extraction methods have been developed to obtain polyphenol-rich extracts from cocoa beans.

An exemplary process for extracting cocoa polyphenols from cocoa beans is disclosed in US 8 451 045 B2.

In general, it is desirable to provide highly concentrated polyphenolic extracts in powder form with a degree of purity as high as possible.

In this context, EP 2 071 961 B1 discloses a process of obtaining polyphenol-rich cocoa powder extracts characterized in that they are obtained by the steps of subjecting freshly depulped cocoa seeds to blanching, drying and defatting by means of pressing, stabilizing the defatted product, grinding and subsequent solid-liquid and liquid-liquid extraction and concentration steps.

EP 1 748 700 A1 discloses a process in which a cocoa polyphenol concentrate is obtained by subjecting unfermented cocoa beans to a blanching step, drying the unfermented cocoa beans, subjecting the dried unfermented cocoa beans to a particle size reduction step, extracting polyphenols from the dry unfermented cocoa bean intermediate to give a cocoa polyphenol extract and extracted solids, and concentrating the cocoa polyphenol extract to yield a cocoa polyphenol concentrate, wherein the process further includes a defatting step which is carried out by pressing the cocoa bean material prior to the extracting step.

However, the total polyphenol yield of these processes based on the initial polyphenol content in the starting material leaves room for improvement. For instance, EP 2 071 961 B1 discloses a total polyphenol yield of 11.93%.

Moreover, providing a polyphenol extract which is not only highly concentrated but simultaneously ensures a favourable bioavailability is not trivial. It has been shown that monomeric flavanols are better absorbed from the intestine than polymerized proanthocyanidins, and thus exhibit a higher antioxidant activity *in vivo.* However, liquid-liquid extraction steps may result in an oligomer distribution in favour of higher oligomers with a polymerization degree (DP) of 3 and higher (see e.g. EP 1 913 821 A1). In addition, large number of processing steps - especially those involving input of thermal and/or mechanical energy - may lead to degradation, epimerization and oxidative polymerization of polyphenols (see, for example, J. Alean et al., Journal of Food Engineering 2016, 189, 99-105).

To avoid the use of mechanical pressing for the separation of fat (i.e. cocoa butter) and to minimize the thermal burden exerted on the cocoa bean material, alternative cocoa bean processing methods have been developed in order to preserve higher contents of valuable heat-sensitive components.

For instance, WO 2010/073117 A1, EP 3 114 940 A1, EP 3 114 941 A1, EP 3 114 942 A1 and EP 3 114 939 A1 disclose methods for processing cocoa beans comprising the formation of a suspension comprising cocoa beans or nibs and water, wet grinding the suspended beans or nibs, heating the suspension, and decanting the same such that said suspension is separated into a water phase, a fat phase and a solid phase, in order to avoid liquefaction of the cocoa fat and formation of a chocolate liqueur during mechanical processing, which exerts lower thermal burden on the cocoa mass compared to conventional methods and hence preserves higher contents of polyphenols, antioxidants and/or vitamins.

Advantageously, these methods envisage manufacture of chocolate products by use of a chocolate construction kit, which includes cocoa butter, cocoa powder, cocoa aroma and polyphenolic powder extracts, so that polyphenol extraction may be fully integrated into a chocolate production process. Herein, the polyphenolic powder is obtained by subjecting the water phase to a dearomatization step, a concentration step (typically an evaporation of excessive water) and optionally spray drying. However, it has been found that these methods are not suitable to provide a highly enriched polyphenolic powder with a total flavanol content of higher than 10 g/100 g of defatted dry matter, as they typically lead to contents of 2 to 8 g/100 of defatted dry matter depending on the cultivar.

In view of the above, it remains desirable to provide a method which provides an improved yield of total polyphenols based on the total polyphenol content in the cocoa fruit constituents, and which produces a polyphenol extract having simultaneously a high concentration of flavanols and a high ratio of monomeric and dimeric flavanols.

Beside of polyphenols, cocoa and cocoa products also contain high amounts of methylxanthines, namely theobromine - known to be a brain stimulant, to have diuretic action, and being a potential for blood pressure reduction - and caffeine, which represent about 2.4-3.6% of defatted dry cocoa composition. Commonly used methods of extracting theobromine from cocoa beans often include extraction with chloroalkanes (e.g. chloroform or dichloromethane), as shown in US 1,925,326 A, for example. However, the use of such extraction solvents is not recommended, both from an environmental perspective and due to the fact that residues of such solvents in a food product are undesirable and require extensive removal steps.

Therefore, it would be desirable to efficiently extract such constituents to simultaneously provide additional extracts in the value chain of cocoa bean processing for the use in the preparation of food compositions, food supplements, medical compositions or cosmetic compositions.

### SUMMARY OF THE INVENTION

The present invention solves this object with the subject matter of the claims as defined herein. The advantages of the present invention will be further explained in detail in the sections below and further advantages will become apparent to the skilled artisan upon consideration of the invention disclosure.

In one aspect, the present invention relates to a method for obtaining extracts from cocoa fruit constituents comprising the steps of: a) subjecting cocoa fruit constituents to wet grinding in a polar solvent and separating a liquid phase from the wet ground mixture to obtain a hydrophilic extract; b) optionally separating residual solids from the hydrophilic extract; c) adsorbing the hydrophilic extract on a non-ionic macroporous resin; d) desorbing the hydrophilic extract from the non-ionic macroporous resin with a water-miscible organic solvent to obtain a polyphenolic extract; and e) concentrating the polyphenolic extract to obtain a polyphenolic concentrate.

In another aspect, the present invention relates to a polyphenolic extract obtained by the aforementioned method, preferably satisfying at least one of the following features: a total flavanol content determined by HPLC of preferably at least 25 g/100 g of defatted dry matter, more preferably at least 30 g/100 g of defatted dry matter, further preferably at least 50 g/100 g of defatted dry matter, especially preferably at least 53 g/100 g of defatted dry matter; a content of monomeric flavanols (DP1) determined by HPLC of at least 18% by weight, preferably at least 20% by weight, more preferably at least 24% by weight, based on the total content of flavanols with a degree of polymerization from 1 to 7 (DP1 to DP7); and a protein content determined by HPLC of less than 42 g/100 g of defatted dry matter, a carbohydrate content determined by HPLC of less than 6 g/100 g of defatted dry matter, and a dietary fiber content determined by HPLC of less than 2 g/100 g of defatted dry matter.

In a further aspect, the present invention relates to a protein-enriched extract obtained by the aforementioned method, wherein the residual solids are further processed by at least a concentration step.

In yet another aspect, the present invention relates to a protein-enriched extract obtained by the aforementioned method, wherein the non-adsorbed fraction of the hydrophilic extract in step c) is further processed by at least a concentration step.

In further aspects, the present invention relates to food compositions, food supplements, medical compositions or cosmetic compositions comprising the aforementioned extracts.

### BRIEF DESCRIPTION OF THE DRAWINGS

FIG. 1 illustrates an exemplary method for obtaining extracts from cocoa fruit constituents according to the present invention.
FIG. 2 schematically shows an exemplary method for the manufacturing of chocolate, making use of the extracts provided by the present invention.

### DETAILED DESCRIPTION OF THE INVENTION

### Extraction and Fractionation Methods

In a first embodiment, the present invention relates to a method for obtaining extracts from cocoa fruit constituents comprising the steps of: a) subjecting cocoa fruit constituents to wet grinding in a polar solvent and separating a liquid phase from the wet ground mixture to obtain a hydrophilic extract; b) optionally separating residual solids from the hydrophilic extract; c) adsorbing the hydrophilic extract on a non-ionic macroporous resin; d) desorbing the hydrophilic extract from the non-ionic macroporous resin with a water-miscible organic solvent to obtain a polyphenolic extract; and e) concentrating the polyphenolic extract to obtain a polyphenolic concentrate.

It has been surprisingly found that the sequence of processing steps a) to e) provides an improved yield of total polyphenols based on the total polyphenol content in the cocoa fruit constituents and produces a polyphenol extract having simultaneously a high concentration of flavanols and a high ratio of monomeric and dimeric flavanols. In addition, since the method does not require the use of food-incompatible organic solvents (such as hexane or haloalkanes, e.g. chloroform, dichloromethane or tetrachloromethane), it may be directly integrated into the preparation of cocoa products suitable for consumption (e.g., chocolate, cocoa beverages, cocoa-based desserts, food supplements or the like) while minimizing the necessary processing steps.

The expression "cocoa fruit constituents", as used herein, denotes one or more constituents of the fruit pod of *Theobroma* cacao, wherein typically about 30 to 50 fruit seeds (i.e. cocoa beans) surrounded by pulp and mucilage are enclosed in a cocoa pod shell. Accordingly, such cocoa fruit constituents may include cocoa beans, cocoa bean shell (CBS) material, cocoa pulp/mucilage and/or cocoa pod shell material. In preferred embodiments of the present invention, the cocoa fruit constituents subjected to step a) comprise cocoa beans, which may also include cocoa nibs.

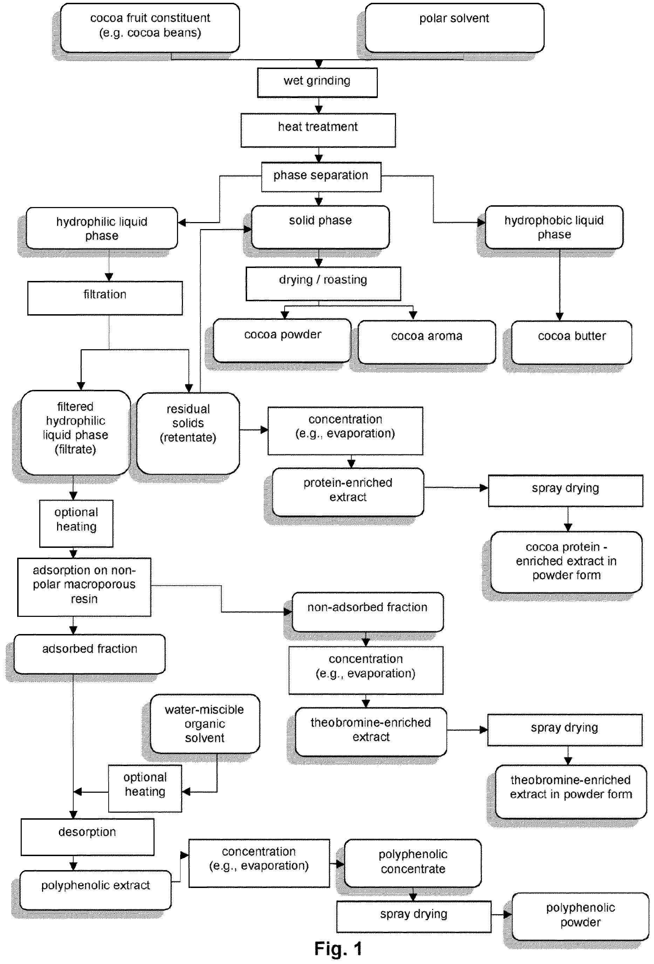

A non-limiting example of the method of the present invention is illustrated in the flowchart shown in Fig. 1.

For the purpose of the present invention, cocoa beans or nibs may be fermented or unfermented, dried or non-dried, roasted or non-roasted, and/or depulped or non-depulped.

However, the total polyphenol content of the defatted dry matter of fresh cocoa beans is typically around 12-20% and of fermented beans approximately 5%. In general, the polyphenol content in cocoa beans depends on their origin and processing (fermentation, drying treatments, alkalization, roasting, etc.), which may substantially affect the polyphenol content and consequently the end product quality. Since up to 70-80% of the polyphenols can be degraded during a fermentation process (see e.g. US 2004/0096566 A1) the cocoa fruit constituents subjected to step a) preferably comprise or consist of unfermented cocoa beans. The expression "fermentation" generally refers to any activity or process involving enzymatic or metabolic decomposition (digestion) of organic materials by microorganisms, and encompasses both anaerobic and aerobic processes, as well as processes involving a combination or succession of one or more anaerobic and/or aerobic stages. The term "unfermented", as used herein, refers to the fermentation degree of the cocoa beans. As is known by those skilled in the art, the degree of fermentation is denoted by the fermentation index (cf. Shamsuddin and Dimick, Qualitative and Quantitative Measurements of Cacao Bean Fermentation, in "Proceedings of the Cacao Biotechnology Symposium", Ed. P. S. Dimick, p. 55-74, The Pennsylvania State University, 1986). In general, the fermentation index of unfermented beans is less than 0.5. It may be further preferred to subject cocoa beans to step a) immediately upon opening the cocoa fruit pod and separating the beans from the pod shell and optionally removing the pulp/mucilage.

Furthermore, polyphenols are generally known as heat-sensitive constituents. Thus, in order to preserve as much of the original polyphenol content of the cocoa beans, the cocoa fruit constituents subjected to step a) preferably comprise cocoa beans which have not undergone a drying and/or roasting process.

In a further preferred embodiment, the unfermented cocoa beans may be pre-treated in an incubation step with or without cocoa pulp and mucilage prior to step (a). Such an incubation step is characterized in that it inhibits the germination of cocoa beans by certain physical and/or chemical pre-treatments shortly after removal of the beans (with or without pulp and mucilage) from the pods.

In particular, it is possible to inhibit germination of cocoa beans by incubating freshly harvested cocoa beans at an elevated temperature, e.g. at a temperature of between 10 and 70 °C, preferably at a temperature of between 10 and 55°C for a period of between 2 hours and 10 days, preferably between 3 and 168 hours, in an incubation medium. As will be known to the skilled artisan, it is particularly preferable to transfer the cocoa beans (optionally together with the pulp/mucilage) to the incubation medium immediately after opening the pod, preferably under sterile conditions, in order to suppress spontaneous fermentation as effectively as possible. The incubation medium is not specifically limited and may be an aqueous acidic medium, as is e.g. used in the cocoa bean processing method disclosed in US 8,501256 B2, or an aqueous ethanol solution, for example.

Furthermore, polyphenols are generally known as heat-sensitive constituents. Thus, in order to preserve as much of the original polyphenol content of the cocoa beans, the cocoa fruit constituents subjected to step a) preferably comprise cocoa beans which have not undergone a drying and/or roasting process, which involves a heat treatment above 100°C or preferably above 70°C.

In step a), cocoa fruit constituents are subjected to wet grinding in a polar solvent and a liquid phase is separated from the wet ground mixture to obtain a hydrophilic extract.

Polar solvents, as used herein, generally have a dielectric constant of 15 or higher, preferably 50 or higher, and more preferably 80 or higher. While polar solvents may include polar protic solvents and polar aprotic solvents, polar protic solvents are preferably used. In preferred embodiments, the polar solvent is selected from water, C₁-C₈ alcohols (e.g. methanol, ethanol, isopropanol), C₂-C₈ ketones (e.g. acetone), C₃-C₇ esters (e.g., methyl acetate, ethyl acetate), C₂-C₈ ethers (e.g., diethylether, methyl t-butyl ether), C₄-C₁₀ lactates, and mixtures thereof. Particularly preferred are water or ethanolic water mixtures. It is further preferred that the polar solvent does not contain halogenated hydrocarbons.

In a preferred embodiment, the polar solvent includes or consists of water. In other preferred embodiments, the polar solvent may comprise water at a content of less than 95% by weight (e.g. less than 90% by weight, less than 80% by weight, less than 50% by weight, less than 30% by weight, less than 10% by weight or less than 5% by weight) admixed with one or more organic solvents which may be miscible or immiscible with water under the extraction conditions. It will be understood that alternative water-containing liquids may also be used as a source of water, preferably liquids selected from one or more of coffee, tea and liquids having water contents of from 60 to about 95 % by weight, such as fruit juices, fruit juice concentrates, or milk, for example.

In preferred embodiments, the polar solvent may comprise an acidic component (including, but not limited to acetic acid, citric acid, or other acids) that is provided in addition to acetic and/or citric acid formed by microbial activity in the beans, which may promote efficient extraction at low temperatures and/or reduce oxidative degradation of polyphenols.

According to the present invention, the cocoa bean constituents are wet-ground in the presence of the polar solvent to avoid or reduce destruction of cellular compartments of the raw cocoa materials due to high mechanical loads or shear stresses involved with dry grinding methods conventionally employed in the art.

While not being particularly limited, the weight ratio of polar solvent to cocoa bean constituents in the suspension subjected to wet-grinding is preferably between 1:1 to 6:1, more preferably 2:1 to 4:1, especially preferably about 3:1, which may advantageously affect the processability in the further steps (e.g. facilitated pumping, grinding and easier phase separation).

It is preferred to wet-grind the cocoa fruit constituents by means of a single or multiple wet grinding step(s) to an average particle size of 100 µm or smaller, preferably 50 µm or smaller, and more preferably 20 µm or smaller. Reducing the cocoa fruit constituents to such a size range substantially increases the exposed surface area of the particle material, therefore allowing it to be more efficiently wetted for improved extraction of polyphenols. The particle size reduction may be accomplished by using disc mills (e.g. perforated disc mill), colloid mills (e.g. toothed colloid mills), or corundum stone mills, for example.

It is preferable that in at least one grinding step, cocoa fruit constituents (e.g. cocoa beans or nibs) are macerated to enable the polar solvent to wet the solids better due to increased available surface area of the macerated solids. The methods and devices used for wet milling are not particularly limited as long as undesirable emulsification by significant frictional heat production or high mechanical forces is avoided. For example, when using multiple grinding steps, a coarse wet grinding step (e.g., optionally with further polar solvent) may be carried out using a perforated disc mill, and the coarsely milled suspension may be pumped to a toothed colloid mill for a fine grinding step. It is understood that if multiple grinding steps are employed, the polar solvent may be modified or altered between the steps (e.g. by addition of other solvents or acids).

In a preferred embodiment of the present invention, step a) comprises the steps of: a1) adding a polar solvent to cocoa fruit constituents to form a suspension; a2) wet grinding said suspension; a3) subjecting said suspension to a heat treatment at a temperature of 70°C or less; and a4) separating the suspension into a hydrophilic liquid phase (heavy phase), a hydrophobic liquid phase (light phase) and a solid phase, said hydrophilic liquid phase comprising the hydrophilic extract as major components and residual solids as minor component, said hydrophobic liquid phase comprising cocoa butter as a major component and solids and/or hydrophilic solvent as minor components, and said solid phase comprising cocoa powder and hydrophilic solvent. Steps a1) and a2) may be carried out on the basis of the preparation and grinding of a suspension according to the description above. In step a3), the wet-ground suspension is subjected to a thermal treatment at a temperature of no more than approximately 70°C in order to reduce the overall thermal load and prevent emulsification of cocoa bean material. From the viewpoint of a favorable balance of cocoa butter yield and preservation of desirable flavors, such as aromatics, anti-oxidants and/or vitamins, heating temperatures of from 43 to 65°C are preferable. In terms of cocoa butter liquefaction and/or improved mechanical phase separation, a heating temperature range of from 45 to 50°C is particularly preferable. Without being limited thereto, heating of the wet-milled suspension may be carried out by a scrap or tube heat exchanger.

Thereafter, in step a4), the suspension is separated into a hydrophilic liquid phase (heavy phase), a hydrophobic liquid phase (light phase) and a solid phase, said hydrophilic liquid phase comprising the hydrophilic extract as major components and residual solids as minor component, said hydrophobic liquid phase comprising cocoa butter as a major component and solids and/or hydrophilic solvent as minor components, and said solid phase comprising cocoa powder and hydrophilic solvent. In addition, the solid phase may comprise residual cocoa butter in a content of up to 30% by weight, preferably less than 27% by weight, more preferably less than 20% by weight relative to the total dry weight.

Preferably, devices employing centrifugal forces may be utilized to achieve mechanical particle separations, such as decanters or nozzle separators. For instance, the suspension may be decanted to separate coarse or large or high mass solids from liquid(s) and then smaller and/or fine solid particles may be further separated from liquids and/or oil products may be separated from non-oil products.

Multiple phase separation and recombination steps may be employed to achieve an improved separation between the hydrophilic liquid phase (heavy phase), the hydrophobic liquid phase (light phase) and the solid phase. For example, the hydrophobic liquid phase obtained by an initial decanting step may be further filtered or centrifuged to separate remaining fine particles or water from the hydrophobic liquid phase and the thus obtained fine particles and polar solvent may be recombined with the polar solvent and solid phases from the initial decanting step or at a later processing stage of said phases.

Upon separation of the three phases, these may be independently processed to separate cocoa butter (from the hydrophobic liquid phase), cocoa powder (from the solid phase) and cocoa aroma (from any of the three phases), for example. While not being limited thereto, examples of independent processing of the phases and their recombination are disclosed in WO 2010/073117 A1, EP 3 114 940 A1, EP 3 114 941 A1, EP 3 114 942 A1, EP 3 114 939 A1, EP 3 747 277 A1, EP 3 747 275 A1 and EP 3 747 276 A1.

According to the present invention, the hydrophilic liquid phase obtained in step a) is further processed to extract a polyphenolic concentrate.

At this stage, if acids were added during step a), they may be optionally removed from the hydrophilic liquid phase or neutralized. Suitable methods of removal are not particularly limited and may be carried out by any suitable method known in the art (e.g. by extractive distillation, reactive distillation, extraction (e.g. liquid-liquid) extraction, emulsion-type liquid membrane processes, salting out or combinations thereof).

Typically, the hydrophilic liquid phase obtained in step a) (or step a4), respectively) comprises residual amounts of solids and cocoa butter. According to the present invention, it is preferred to separate residual solids from the hydrophilic extract in order to remove fine particles and cocoa butter residues which may adversely affect the adsorption process in step c). For this purpose, it is preferred to subject the hydrophilic liquid phase to a filtration step in step b).

In a particularly preferred embodiment, cross-flow filtration and/or filtration through membrane filters having an absolute pore size rating of 0.45 µm or less is used. It is especially preferred to employ a dynamic cross-flow filtration using a ceramic membrane, where the cross-flow effect (= tangential overflow of the membrane surface) is generated by rotating the filter discs and not by pumping large volumes of liquid. Hence, efficient filtration may be carried out continuously at relatively high solids loads without filter clogging. It is further preferred to use membrane filters having an absolute pore size rating of 0.30 µm or less, such as 0.25 or less or 0.22 µm or less. In some embodiments, the use of membrane filters having an absolute pore size rating of 0.20 µm or less may be particularly preferred. For instance, it has been found that filtration with a membrane filter having a pore size rating between 0.06 µm and 0.16 µm favourably enables higher flavonoid concentrations in the desorbed polyphenolic extract without substantially decreasing the yield of flavonoids.

In preferred embodiments, the filtration may be carried out at a temperature of from 3 to 50°C, preferably between 25 and 35°C.

In particularly preferred embodiments, a diafiltration step is further integrated to promote flavonoid extraction by introducing purified solvent (e.g. nanofiltered (NF) or reverse osmosis (RO) water) to the process fluid.

The retentate obtained upon filtration (i.e. residual solids) may be further processed by at least a concentration step to obtain a protein-enriched extract (optionally further comprising a large fraction of dietary fibers), which may be brought about by evaporation of residual polar solvent in one or multiple steps. Optionally, the thus obtained protein-enriched extract may be subjected to spray drying to provide a cocoa-based, protein-enriched powder, which represents an additional valuable and previously often discarded constituent.

The permeate (i.e. cleared hydrophilic extract) obtained in the filtration step typically contains cocoa butter in an amount of less than 1 g/100 g dry matter. The major fraction of flavanols and theobromine contained in the hydrophilic phase is generally also still found in the permeate, together with a large amount of amino acids.

In step c), the thus obtained hydrophilic extract may be optionally gently heated (below 70°C) and adsorbed on a non-ionic macroporous resin. For this purpose, a column comprising the non-ionic macroporous resin is prepared, washed and suitably conditioned by methods known to the skilled artisan, and the hydrophilic extract is pumped through the column, whereupon the cocoa polyphenols are adsorbed on the resin.

In a preferred embodiment, the non-ionic macroporous resin comprises or consists of a crosslinked aliphatic polymer.

In terms of improved binding capacity and process efficiency, the non-ionic macroporous resin preferably has a surface area determined by a nitrogen BET method of at least 400 m²/g, more preferably at least 450 m²/g and further preferably at least 500 m²/g. Alternatively or in combination, it is preferred that the non-ionic macroporous resin has a total pore volume determined by a nitrogen BET method of at least 0.80 cc/g, more preferably at least 0.85 cc/g, and further preferably at least 0.90 cc/g.

Upon adsorption, the resin may be optionally washed (e.g. with deionized water) to remove undesired constituents. The washing step is preferably carried out at a temperature of from 10 to 50°C. Notably, the non-adsorbed fraction of the hydrophilic extract in step c) may be further processed by at least a concentration step to obtain a theobromine-enriched extract.

In step d), the hydrophilic extract is desorbed from the non-ionic macroporous resin by elution of adsorbate with a water-miscible organic solvent to obtain a polyphenolic extract.

The water-miscible organic solvent is preferably selected from C₁-C₈ alcohols, C₃-C₅ ketones, C₃-C₅ esters, C₂-C₄ ethers, C₂-C₅ aldehydes and mixtures thereof. Especially preferred water-miscible organic solvents include methanol, ethanol, isopropanol and/or acetone. Optionally, the water-miscible organic solvent may be diluted with water, provided that the water content does not exceed 50 vol.-%. In a particularly preferred embodiment, an aqueous ethanolic solution (e.g. 60-80 vol.-% ethanol) may be used. It is further preferred that the water-miscible organic solvent does not contain halogenated hydrocarbons.

Notably, the loaded resin may be eluted successively with the same solvents but at different temperatures, or with different solvents. For instance, it may be envisaged that a first solvent adapted for a high solubility for caffeine and/or theobromine is first used to selectively elute the alkaloids from the non-ionic macroporus resin, and a second solvent (i.e. the water-miscible organic solvent) is then employed to elute the polyphenolic constituents. Depending on the desired quality of the individual extracts, the desorption process may be optimized accordingly.

Optionally, the water-miscible organic solvent may be heated prior to elution in order to facilitate removal of undesired volatile compounds.

In step e) the polyphenol extract obtained in step d) is subjected to a concentration step to obtain a polyphenolic concentrate. A concentration may be accomplished by removal of the water-miscible solvent in one or multiple steps, e.g. by evaporation or distillation, preferably under reduced pressure or vacuum conditions.

In order to preserve as many polyphenols contained in the original cocoa fruit constituents as possible, the cocoa fruit constituents and their extracts are preferably not subjected to temperatures above 70°C at least during steps a) to d), more preferably during steps a) to e), and further preferably during the entire extraction process.

Advantageously, the above-described method may be fully integrated into a method for the preparation of cocoa-based products for human consumption (e.g. chocolate, cocoa powder, cocoa butter etc.) without relying upon undesirable solvents and provides an excellent yield of cocoa polyphenols. Specifically, total polyphenol yields of 15% or higher relative to the polyphenol content in the cocoa beans (100%), e.g. in the range of 15 to 30%, typically at least 20%, may be achieved.

### Cocoa Extracts

In a second embodiment, the present invention relates to a polyphenolic extract obtained by the method according to the first embodiment described above.

In general, polyphenolic compounds originally present in cocoa beans mainly include catechins ((-)-epicatechin, (+)-catechin, (+)-gallocatechin and (-)epigallocatechin), procyanidins (procyanidins B1-B5, procyanidin C1, procyanidin D and higher oligo- and polymers, mostly homologues of epicatechin with 2 to 18 monomeric units), anthocyanins (i.e. cyanidin-3α-L-arabinosid and cyanidin-3-β-D-galactosid), flavanol glycosides (i.e. quercetin-3-*O*-α-D-arabinosid and quercetin-3-*O*-β-D-glucopuranosid), clovamide and dideoxyclovamide.

The terms "cocoa flavanols" or "flavanols", as used herein, denotes monomeric flavanols (-)-epicatechin and (+)-catechin and their related oligomeric flavanols (procyanidins) with a degree of polymerization (DP) of from DP1 to DP7. The determination of the individual contents based on the degree or polymerization (DP1 to DP7) and the total flavanol content may be determined by HPLC methods coupled with fluorescence detection using commercially available standards (e.g. according to U. Bussy et al., Food Funct. 2020, 11(1), 131-138).

According to a preferred embodiment, the polyphenolic extract of the present invention exhibits a total flavanol content determined by HPLC of at least 25 g/100 g of defatted dry matter, more preferably at least 30 g/100 g of defatted dry matter, further preferably at least 50 g/100 g of defatted dry matter, and especially preferably at least 53 g/100 g of defatted dry matter. Notably, the total flavanol content may exceed that of currently available flavanol standards (e.g. NIST Reference Material 8403). The upper limit of the total flavanol content is not particularly limited, and may be 100 g/100 g of defatted dry matter.

In addition or alternatively, the polyphenolic extract of the present invention preferably exhibits a content of monomeric flavanols (DP1) determined by HPLC of at least 18% by weight, more preferably at least 20% by weight, and further preferably at least 24% by weight, based on the total content of flavanols with a degree of polymerization from 1 to 7 (DP1 to DP7). It may be further preferred that the polyphenolic extract of the present invention preferably exhibits a content of dimeric flavanols (DP2) determined by HPLC of at least 18% by weight, preferably at least 20% by weight, based on the total content of flavanols with a degree of polymerization from 1 to 7 (DP1 to DP7). Accordingly, the polyphenolic extracts contain high contents of monomeric and dimeric flavanols, which exhibit particularly favourable bioavailability.

In addition or alternatively, the polyphenolic extract of the present invention preferably exhibits a protein content determined by HPLC of less than 42 g/100 g of defatted dry matter (e.g., from 5 to 41 g/100 g of defatted dry matter), a carbohydrate content determined by HPLC of less than 6 g/100 g of defatted dry matter (e.g., from 2 to 5 g/100 g of defatted dry matter), and a dietary fiber content determined by HPLC of less than 2 g/100 g of defatted dry matter (e.g., from 0.1 to 1.8 g/100 g of defatted dry matter). The aforementioned nutrient contents are characteristic features of polyphenolic extracts prepared by the method of the present invention.

In a third embodiment, the present invention relates to a protein-enriched extract obtained by the method according to the first embodiment. As outlined above, the protein-enriched extract is derived from the retentate obtained upon filtration (i.e. residual solids), which is subjected to at least a concentration step which effects the removal of the polar solvent. Optionally, the thus obtained protein-enriched extract may be subjected to spray drying to provide a cocoa-based, protein-enriched powder.

Typically, the protein-enriched powder has a protein content of at least 30% by weight, usually more than 35% by weight based on the total dry weight of proteins, carbohydrates and dietary fibers. The respective contents may be suitably determined by HPLC methods.

In a fourth embodiment, the present invention relates to a theobromine-enriched extract obtained by the method according to the first embodiment. As outlined above, the theobromine-enriched extract is derived from the non-adsorbed fraction of the hydrophilic extract in step c), which is further processed by at least a concentration step. The theobromine-enriched extract typically exhibits a theobromine content of at least 4 g/100 g of defatted dry matter and preferably at least 5 g/100 g of defatted dry matter.

### Compositions and Uses of the Extracts

In a fifth embodiment, the present invention relates to food compositions, food supplements, medical compositions or cosmetic compositions comprising the extracts according to the second to fourth embodiments or comprising the polyphenolic extracts, protein-enriched extracts and/or theobromine-enriched extracts produced according to the first embodiment, respectively.

It will be understood that the individual extracts obtained by the methods of the present invention may be used and/or sold independently or integrated into a cocoa bean processing method directed at the provision of food and beverage compositions.

Exemplary food compositions according to the present invention include liquid and solid cocoa-based products suitable for consumption (e.g., chocolate, cocoa-based desserts, cacao drinks).

In addition, the extracts may be used and provided in terms of a construction kit for cocoa-based products (e.g., chocolate, cocoa-based desserts, cacao drinks) which may further comprise a cocoa powder (including, but not limited to a cocoa powder provided by drying the solid phase obtained in step a) or a4) respectively), cocoa butter (including, but not limited to cocoa butter provided by processing the hydrophobic liquid phase obtained in step a4)) and/or cocoa aroma (including, but not limited to cocoa aroma retrieved by dearomatization of the hydrophilic liquid phase obtained in step a4)).

As an example, it may be envisaged to employ the polyphenolic extracts, protein-enriched extracts and/or theobromine-enriched extracts in the methods of WO 2010/073117 A1, EP 3 114 940 A1, EP 3 114 941 A1, EP 3 114 942 A1, EP 3 114 939 A1, EP 3 747 277 A1, EP 3 747 275 A1 and EP 3 747 276 A1, and thereby enable a further fine-tuning of the organoleptic and/or nutritional properties of the desired products.

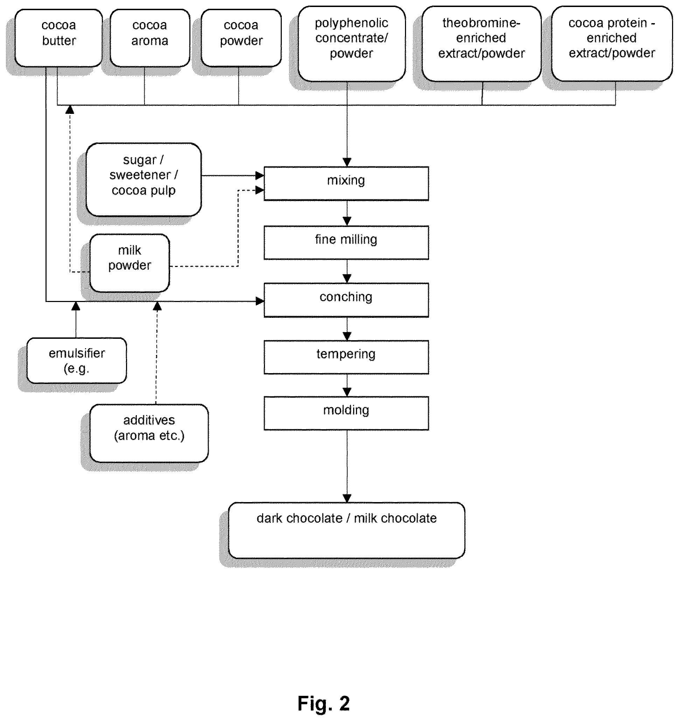

Fig. 2 illustrates an example of a method for the preparation of dark chocolate and milk chocolate based on a chocolate construction kit comprising cocoa aroma, cocoa powder, cocoa butter, and the polyphenolic extract, protein-enriched extract and theobromine-enriched extract produced according to the present invention. While not being limited thereto, cocoa aroma may be obtained from dearomatization of the hydrophilic phase and/or may be roasted cocoa aroma obtained from drying/roasting of cocoa powder. Before being subjected to a conching step, the kit constituents are mixed. The polyphenolic extract according to the present invention may be used to enhance the yield of polyphenols when compared to the prior art processes and improve the bioavailability of the final products. The protein-enriched extract, a previously unused fraction of the abovementioned prior art cocoa bean processing methods, may be added or re-introduced to modify the protein content in the final products and make ideal and effective use of the cocoa bean constituents. Additional tailoring of flavor or development of flavor may be performed by adding one or more of sugar, sweetener, cocoa pulp and/or fruit juices. In addition, the theobromine-enriched extract may be selectively added or re-introduced in order to make use of the pharmacological effects of the alkaloid(s) and/or to balance the bitterness of the cocoa-based product.

For the preparation of milk chocolate, milk powder is further added, preferably prior to the mixing step. Optionally, an emulsifying agent (e.g. lecithin) may be added prior to conching to reduce viscosity, control sugar crystallization and the flow properties of chocolate, and help in the homogeneous mixing of ingredients. Also, additional ingredients and flavors, such as e.g. vanilla, rum or the like may be added prior to or during the conching step, and after conching (e.g. sea salt, fleur de sel, nuts and raisins). The conching process redistributes into the fat phase the substances from the dry cocoa that create flavor, while removing unwanted acetic, propionic, and butyric acids from the chocolate, reducing moisture, and mellowing the flavor of the product. The temperature of the conche is controlled and varies depending on the different types of chocolate (from around 49 °C for milk chocolate to up to 82 °C for dark chocolate). While being to some degree dependent on the temperature, the conching duration in conventional chocolate manufacturing processes generally ranges from 16 up to 72 hours in order to achieve good results. The conching duration is preferably less than 16 hours, more preferably less than 12 hours, typically 10 hours or less. Thus, a loss of desirable aroma characteristics and the degradation of heat-sensitive components (including the polyphenolic fractio) is minimized.

For the purpose of a medical composition or dietary supplement, the extracts according to the present invention may be suitably administered in a form (e.g. in capsules or tablets) and dose suitably selected by a skilled artisan.

Due to the antioxidant properties of the polyphenolic constituents, the polyphenolic concentrate obtained by the present invention may also be employed in cosmetic applications, e.g. in shampoos, lotions, creams, balms, ointments, or the like.

It will be understood that the features of the first to fifth embodiments may be freely combined in any combination, except for combinations where at least some of the features are mutually exclusive.

### EXAMPLES

### Extraction and Phase Separation

2569 kg of fermented, debacterized and non-roasted cocoa nibs were combined with water as a polar solvent in a ratio of approximately 1:3 to form a suspension. Subsequently, said suspension was wet ground in multiple stages using a perforated colloid mill to an average particle size of less than 100 µm and heated to a temperature of about 45 to 60°C via a tube heat exchanger. Using a three-phase decanter, the two non-mixable liquid phases (i.e. the hydrophilic liquid phase and the hydrophobic liquid phase) were separated from each other with a simultaneous discharge of the solid phase. Upon removal of the lighter oil phase and the solid phase, the hydrophilic liquid phase was used to provide polyphenol extracts, theobromine-enriched extracts and protein-enriched extracts according to the procedures described in the following sections.

### Filtration

Initially, the hydrophilic extract was subjected to a filtration step using a dynamic cross-flow filtration system with ceramic membrane filter discs (commercially available by Novoflow GmbH; filter area: 2 m²; membrane pore size: 0.2 µm) at a temperature of 5-10 °C. A diafiltration was performed by introducing reverse-osmosis water at a volume of 10% relative to the total volume of the hydrophilic extract feed.

Two products were obtained from the filtration of the aqueous cocoa extract, the retentate, which mainly comprises proteins and dietary fibers, and on the other hand the filtrate, which mainly contains aqueous solvent, cocoa flavanols, caffeine and theobromine next to proteins. The retentate was then concentrated in an evaporator and converted into a powder by spray drying.

Furthermore, as is shown in Table 1, the filtration step effectively removed residual fats (i.e. cocoa butter) from the hydrophilic extract to avoid an interference with the subsequent adsorption.

**TABLE 1: Mass balance of cocoa butter over the extraction and filtration process. The calculation of the mass balance percentage is based relative to the percentage of 100% attributed to the cocoa nibs.**

| Process | Sample | | Content | Mass balance | |
|---|---|---|---|---|---|
| | | | [g/100 g dry mass] | [kg] | [%] |
| Extraction | cocoa nibs | | 54.51 | 1328.69 | 100.00 |
| | solid phase | | 24.08 | 241.53 | 18.18 |
| | liquid phase | hydrophobic | 100.00 | 950.00 | 71.5 |
| | | hydrophilic | 10.96 | 28.76 | 2.16 |
| Filtration | retentate | 30.57 | 30.73 | 2.31 | |
| | permeate (filtrate) | 0.43 | 0.62 | 0.05 | |

In addition to reducing the fat content in the cocoa extract, the distribution of bioactive alkaloids theobromine and caffeine was monitored via HPLC, the results being shown in Tables 2 and 3.

**TABLE 2: Mass balance of alkaloids (caffeine and theobromine) over the extraction and filtration process. The calculation of the percentage balance refers to the cocoa nibs, which are assumed to be 100%.**

| Process | Sample | Caffeine | | Theobromine | |
|---|---|---|---|---|---|
| | | [kg] | [%] | [kg] | [%] |
| Extraction | cocoa nibs | 522 | 100 | 2877 | 100 |
| | solid phase | 165 | 32 | 865 | 30 |
| | hydrophilic liquid phase | 253 | 49 | 1350 | 47 |
| Filtration | retentate | 56 | 11 | 51 | 10 |
| | permeate (filtrate) | 118 | 23 | 815 | 28 |

**TABLE 3: Content of alkaloids (caffeine and theobromine) in the individual intermediate products from the extraction and filtration process, determined via HPLC.**

| Process | Sample | Caffeine | Theobromine |
|---|---|---|---|
| | | [g/100 g defatted dry mass] | [g/100 g defatted dry mass] |
| Extraction | cocoa nibs | 0.47 | 2.59 |
| | solid phase | 0.22 | 1.14 |
| | hydrophilic liquid phase | 1.11 | 5.92 |
| Filtration | retentate | 0.81 | 4.21 |
| | permeate (filtrate) | 0.83 | 5.74 |

Table 3 demonstrates that during the extraction process, the alkaloids were enriched in the hydrophilic liquid phase (1.11 g/100 g caffeine and 5.92 g/100 g theobromine based on the defatted dry matter). As illustrated by the mass balance, the alkaloids permeated well through the filtration membrane.

The balance of the cocoa flavanols (DP1-DP7) over the extraction and filtration process was analyzed via HPLC and fluorescence detection.

The results are summarized in Table 4, and show that the flavanols with a low degree of polymerization (DP1-DP3) in particular could be extracted well with the aqueous solvent, whereas the flavanols with a higher degree of polymerization (i.e. DP4-DP7) are predominantly found in the solid phase.

Similarly, in the filtration process approximately 25-37% of the flavanols DP1-DP4 of the initial content in the cacao nibs are recovered the permeate, whereas the yield of the flavanols DP5-DP7 decreases sharply at 8-15%.

**TABLE 4: Mass balance of cocoa flavanols (DP1-DP7) over the extraction and filtration processes, determined via HPLC/fluorescence detection.**

| Process | Sample | DP1 | | DP2 | | DP3 | | DP4 | | DP5 | | DP6 | | DP7 | | Total | |
|---|---|---|---|---|---|---|---|---|---|---|---|---|---|---|---|---|---|
| | | [kg] | [%] | [kg] | [%] | [kg] | [%] | [kg] | [%] | [kg] | [%] | [kg] | [%] | [kg] | [%] | [kg] | [%] |
| Extraction | cocoa nibs | 917 | 100 | 781 | 100 | 886 | 100 | 714 | 100 | 596 | 100 | 442 | 100 | 306 | 100 | 4650 | 100 |
| | solid phase | 312 | 34 | 303 | 39 | 375 | 42 | 340 | 48 | 334 | 56 | 289 | 65 | 239 | 78 | 2190 | 47 |
| | hydrophilic liquid phase | 450 | 49 | 384 | 49 | 372 | 42 | 266 | 37 | 182 | 31 | 114 | 26 | 68 | 22 | 1838 | 40 |
| Filtration | permeate (filtrate) | 339 | 37 | 291 | 37 | 271 | 31 | 180 | 25 | 99 | 17 | 48 | 11 | 25 | 8 | 1253 | 27 |

Table 5 lists the contents of cocoa flavanols in the fat-free dry matter of the individual intermediate products. It is shown that the cocoa flavanols are predominantly found in the hydrophilic liquid phase and were effectively recovered and enriched in the permeate after the filtration step. In this experiment, the total yield of cocoa flavanols in the permeate (filtrate) was 27% based on the total cocoa flavanol content in the cocoa nibs (assumed as 100%).

**TABLE 5: Total content of cocoa flavanols (DP1-DP7), determined via HPLC/fluorescence detection.**

| Process | Sample | Total content of cocoa flavanols DP1-DP7 |
|---|---|---|
| | | [g/100 g defatted dry mass] |
| Extraction | cocoa nibs | 4.19 |
| | solid phase | 2.88 |
| | hydrophilic liquid phase | 7.87 |
| Filtration | retentate | 0.00 |
| | permeate (filtrate) | 8.83 |

Table 6 shows the balance of nutritional values (carbohydrates, dietary fibers, proteins) in the individual intermediate products over the extraction and filtration process. The calculation of the percentage balance refers to the cocoa nibs, which are assumed to be 100%.

**TABLE 6: Relative contents of nutrients (carbohydrates, dietary fibers, proteins) in the individual intermediate products over the extraction and filtration process, determined via HPLC.**

| Process | Sample | Carbohydrates | Dietary Fibers | Proteins |
|---|---|---|---|---|
| | | [%] | [%] | [%] |
| Extraction | cocoa nibs | 100 | 100 | 100 |
| | hydrophilic liquid phase | 13 | 15 | 30 |
| Filtration | retentate | 5 | 6 | 7 |
| | permeate (filtrate) | 12 | 6 | 19 |

It is shown that during the extraction process, the main part of carbohydrates, dietary fibers and proteins is not extracted by water and are predominantly retained in the solid phase. In the filtration step, carbohydrates and proteins predominantly permeate into the filtrate, whereas dietary fibers are evenly distributed between the retentate and filtrate. A protein yield of 19% based on the initial content in the cacao nibs is found in the permeate.

### Adsorption/desorption of cocoa flavanols

After the filtration step, the permeate (filtrate) was subjected to an adsorption/desorption step. For this purpose, glass columns were made and filled with a polymeric adsorbent (AmberLite^{™} XAD^{™}7HP, commercially available by DuPont^{™}). The adsorbent used was a non-ionic, aliphatic acrylic resin, having a surface area of at about 520 m²/g and a total pore volume of about 0.95 cc/g, each determined by a nitrogen BET method. After washing and conditioning the adsorbent with deionized water, the permeate was pumped into the column and thus contacted with the non-ionic macroporous resin. Upon loading, the column was washed with deionized water. Thereafter, the product was desorbed from the non-ionic macroporous resin elution with a hydroalcoholic solution with a polar gradient (60-80% ethanol). As a result, the cocoa flavanols were released from the adsorbent and can be pumped out of the column with the solvent to provide a polyphenolic extract. The enriched polyphenol fraction obtained was then concentrated in an evaporator and processed by means of spray drying to provide a polyphenolic concentrate in powder form. Similarly, the non-adsorbed fraction was collected, concentrated in an evaporator and processed into a powder by means of spray drying to provide the theobromine-enriched extract.

In analogy to the above measurements for the extraction and filtration steps, the individual fractions were analyzed for their contents of alkaloids, flavanols and nutrients.

Table 7 shows the yield and distribution of the alkaloids caffeine and theobromine in the individual intermediate and end products over the entire three processing stages.

**TABLE 7: Relative mass balance of alkaloids (caffeine and theobromine) over the stages extraction, filtration and adsorption/desorption. The calculation of the percentage balance refers to the cocoa nibs, which are assumed to be 100%.**

| Process | Sample | Caffeine | Theobromine |
|---|---|---|---|
| | | [%] | [%] |
| Extraction | cocoa nibs | 100 | 100 |
| | solid phase | 32 | 30 |
| | hydrophilic liquid phase | 49 | 47 |
| Filtration | retentate | 11 | 10 |
| | permeate (filtrate) | 23 | 28 |
| Adsorption/Desorption | theobromine-enriched extract | 11 | 11 |
| | polyphenolic concentrate | 12 | 6 |

The results show that while theobromine is predominantly found in the corresponding enriched extract, caffeine is distributed almost equally between the theobromine-enriched extract and the polyphenolic concentrate. The concentration of theobromine in the theobromine-enriched extract was determined as 4.5 g/100 g of defatted dry mass.

Table 8 shows the content and distribution of cocoa flavanols (DP1-DP7) throughout the adsorption/desorption process relative to the permeate obtained in the filtration step. It is shown that 75% of the initial flavanol content could be recovered in the polyphenol concentrate, while simultaneously reducing the ratio of higher oligomers (DP6 and DP7), which are predominantly found in the theobromine-enriched extract. A favourably high content of monomeric flavanols (DP1) of 12.9 g/100g of defatted dry matter was achieved, corresponding to a content of 24.2 % by weight based on the total content of flavanols with a degree of polymerization from 1 to 7 (DP1 to DP7).

**TABLE 8: Content distribution and yield of cocoa flavanols (DP1-DP7) after adsorption/desorption as determined via HPLC/fluorescence detection, relative to the contents in the permeate (filtrate) assumed as being 100%.**

| Process | Sample | DP1 | | DP2 | | DP3 | | DP4 | | DP5 | | DP6 | | DP7 | | Total | |
|---|---|---|---|---|---|---|---|---|---|---|---|---|---|---|---|---|---|
| | | [kg] | [%] | [kg] | [%] | [kg] | [%] | [kg] | [%] | [kg] | [%] | [kg] | [%] | [kg] | [%] | [kg] | [%] |
| Filtration | permeate (filtrate) | 339 | 100 | 291 | 100 | 271 | 100 | 180 | 100 | 99 | 100 | 48 | 100 | 25 | 100 | 1253 | 100 |
| Adsorption / Desorption | theobromine-enriched powder | 82 | 24 | 82 | 28 | 22 | 8 | 13 | 7 | 9 | 9 | 69 | 144 | 52 | 210 | 199 | 16 |
| | polyphenolic concentrate | 228 | 67 | 199 | 68 | 221 | 82 | 146 | 81 | 86 | 86 | 42 | 88 | 20 | 81 | 944 | 75 |

Table 9 summarizes the contents of cocoa flavanols in the defatted dry matter of the individual intermediate and final products. The results demonstrate that the process of the present invention provides a polyphenolic extract with a favourably high concentration of cocoa flavanols of 53.55 g/100 g of defatted dry matter.

**TABLE 9: Total content of cocoa flavanols (DP1-DP7), determined via HPLC/fluorescence detection.**

| Process | Sample | Total content of cocoa flavanols DP1-DP7 |
|---|---|---|
| | | [g/100 g of defatted dry matter] |
| Extraction | cocoa nibs | 4.19 |
| | solid phase | 2.88 |
| | hydrophilic liquid phase | 7.87 |
| Filtration | retentate | 0.00 |
| | permeate (filtrate) | 8.83 |
| Adsorption / Desorption | theobromine-enriched powder | 2.75 |
| | polyphenolic concentrate | 53.55 |

The total yield of cocoa flavanols in the polyphenolic concentrate was 20.3% relative to the cocoa flavanol content present in the starting material, i.e. the cocoa nibs. Accordingly, it has been demonstrated that the process of the present invention simultaneously enables the provision of an extract with a high concentration of flavanols, a high ratio of monomeric and dimeric flavanols and in an excellent yield of total polyphenols based on the total polyphenol content in the cocoa fruit constituents.

In order to further characterize the extracts obtained by the process, the relative contents of nutrients (carbohydrates, dietary fibers, proteins) was determined via HPLC. The results are shown in Table 10 in combination with the contents of the intermediate product of the filtration step.

**TABLE 10: Relative contents of carbohydrates, dietary fibers, proteins in the intermediate and final products, as determined via HPLC.**

| Process | Sample | Carbohydrates | Dietary Fibers | Proteins |
|---|---|---|---|---|
| | | [%] | [%] | [%] |
| Extraction | cocoa nibs | 100 | 100 | 100 |
| | hydrophilic liquid phase | 13 | 15 | 30 |
| Filtration | retentate | 5 | 6 | 7 |
| | permeate (filtrate) | 12 | 6 | 19 |
| Adsorption / Desorption | theobromine-enriched powder | 7.9 | 0.9 | 8.9 |
| | polyphenolic concentrate | 0.3 | 0.1 | 2.1 |

The results show that a substantial fraction of nutrients found in the permeate is separated through the non-adsorbed fraction providing the theobromine-enriched extract. In the polyphenolic concentrate, when considering the contents in relation to the defatted dry matter, it is noticeable that the proteins make up the largest part of the nutritional values with 40.1 g/100 g of defatted dry matter, followed by carbohydrates with 4.7 g/100 g of defatted dry matter and dietary fibers with 1.5 g/100 g of defatted dry matter.

Once given the above disclosure, many other features, modifications, and improvements will become apparent to the skilled artisan.

## Claims

1. Method for obtaining extracts from cocoa fruit constituents comprising the steps of:
a) subjecting cocoa fruit constituents to wet grinding in a polar solvent and separating a liquid phase from the wet ground mixture to obtain a hydrophilic extract;
b) optionally separating residual solids from the hydrophilic extract;
c) adsorbing the hydrophilic extract on a non-ionic macroporous resin;
d) desorbing the hydrophilic extract from the non-ionic macroporous resin with a water-miscible organic solvent to obtain a polyphenolic extract; and
e) concentrating the polyphenolic extract to obtain a polyphenolic concentrate.

2. Method according to claim 1, wherein step a) comprises the steps of:
a1) adding a polar solvent to cocoa fruit constituents to form a suspension;
a2) wet grinding said suspension;
a3) subjecting said suspension to a heat treatment at a temperature of 70°C or less; and
a4) separating the suspension into a hydrophilic liquid phase (heavy phase), a hydrophobic liquid phase (light phase) and a solid phase,
said hydrophilic liquid phase comprising the hydrophilic extract as major components and residual solids as minor component, said hydrophobic liquid phase comprising cocoa butter as a major component and solids and/or hydrophilic solvent as minor components, and said solid phase comprising cocoa powder and hydrophilic solvent.

3. Method according to claim 1 or claim 2,
wherein the polar solvent is selected from water, C₁-C₈ alcohols, C₂-C₈ ketones, C₃-C₇ esters, C₂-C₈ ethers, C₄-C₁₀ lactates, and mixtures thereof, and may optionally include an acid, preferably acetic acid or citric acid; and/or
wherein the water-miscible organic solvent is selected from C₁-C₈ alcohols, C₃-C₅ ketones, C₃-C₅ esters, C₂-C₄ ethers, C₂-C₅ aldehydes and mixtures thereof, and preferably from methanol, ethanol, isopropanol or acetone.

4. Method according to any of claims 1 to 3, wherein the cocoa fruit constituents in step a) comprise unfermented and non-roasted cocoa beans, preferably unfermented and non-roasted cocoa beans which have not been subjected to drying.

5. Method according to any of claims 1 to 4, wherein in step a) cocoa fruit constituents are subjected to a single or multiple wet grinding step(s) to an average particle size of 100 µm or smaller, preferably 50 µm or smaller, and more preferably 20 µm or smaller.

6. Method according to any of claims 1 to 5, wherein the cocoa fruit constituents and their extracts are not subjected to temperatures above 70°C.

7. Method according to any of claims 1 to 6, wherein in step e), the polyphenolic concentrate is subjected to a concentration or evaporation step and an optional spray drying step to obtain polyphenolic powder.

8. Method according to any of claims 1 to 7, wherein the non-ionic macroporous resin has a surface area determined by a nitrogen BET method of at least 400 m²/g and/or a total pore volume determined by a nitrogen BET method of at least 0.90 cc/g.

9. Method according to any of claims 1 to 8, wherein in step b), residual solids are separated from the hydrophilic extract by filtration, preferably by cross-flow filtration and/or filtration through membrane filters having an absolute pore size rating of 0.45 µm or less.

10. Method according to claim 9, wherein the residual solids are further processed by at least a concentration step to obtain a protein-enriched extract.

11. Method according to any of claims 1 to 10, wherein the non-adsorbed fraction of the hydrophilic extract in step c) is further processed by at least a concentration step to obtain a theobromine-enriched extract.

12. Polyphenolic extract obtained by the method according to any one of claims 1 to 11, preferably satisfying at least one of the following features:
a total flavanol content determined by HPLC of at least 25 g/100 g of defatted dry matter, preferably at least 30 g/100 g of defatted dry matter, further preferably at least 50 g/100 g of defatted dry matter, especially preferably at least 53 g/100 g of defatted dry matter;
a content of monomeric flavanols (DP1) determined by HPLC of at least 18% by weight, preferably at least 20% by weight, more preferably at least 24% by weight, based on the total content of flavanols with a degree of polymerization from 1 to 7 (DP1 to DP7); and
a protein content determined by HPLC of less than 42 g/100 g of defatted dry matter, a carbohydrate content determined by HPLC of less than 6 g/100 g of defatted dry matter, and a dietary fiber content determined by HPLC of less than 2 g/100 g of defatted dry matter.

13. Protein-enriched extract obtained by the method according to claim 10.

14. Theobromine-enriched extract obtained by the method according to claim 11.

15. Food compositions, food supplements, medical compositions or cosmetic compositions comprising the extracts according to claims 12 to 14.
